# DEMANDE DE BREVET EUROPEEN

(11) **EP 1 151 736 A1**
(43) Date de publication de la demande: **07.11.2001**
(21) Numéro de dépôt: 01420100.8
(22) Date de dépôt: 03.05.2001
(51) Int. Cl.: A61F 13/56

(54) **Couches-culottes à attaches déployables**

(30) Priorité: 04.05.2000 FR 0005714
(71) Demandeur: Prod'hygia S.A., 59494 Petite Foret (FR)
(72) Inventeur: Lorthioit, Bruno, 59171 Hornaing (FR); Comte, Tony, 69220 St Jean d'Ardieres (FR)

(57) **Abrégé**

Il s'agit d'une couche-culotte (1) du type comportant des parties avant et arrière à assembler à l'aide de moyens de fixation pour maintenir la couche en place sur l'utilisateur. Les moyens de fixation comportent au moins une attache (20) apte à être déployée, cette attache étant maintenue initialement par des moyens de maintien dans un état rétracté et comportant une partie de préhension permettant à l'utilisateur de la déployer, les moyens de maintien comportant deux feuilles superposées prenant l'attache (20) en sandwich.

## Description

La présente invention concerne le domaine des articles d'hygiène et plus particulièrement celui des couches-culottes.

On connaît des couches-culottes qui se fixent sur l'utilisateur en assemblant leurs parties arrière et avant à l'aide d'attaches assujetties à la partie arrière de la couche et comportant des éléments de fixation, par exemple à crochets.

Lors de la production et notamment de l'empaquetage des couches-culottes, les attaches sont maintenues rabattues sur la partie arrière par un adhésif pour éviter d'une part qu'elles ne soient endommagées lors de la manutention en machine et pour offrir d'autre part à l'utilisateur un produit d'aspect le plus satisfaisant possible.

Pour permettre le décollement des attaches lors de l'utilisation, des bandes de réception siliconées sont rapportées sur la couche, sur lesquelles l'adhésif des attaches adhère faiblement.

De telles bandes de réception sont relativement coûteuses.

Il existe un besoin notamment pour réduire le coût de fabrication des couches-culottes.

La présente invention y parvient en proposant une couche-culotte du type comportant deux parties avant et arrière à assembler à l'aide de moyens de fixation pour maintenir la couche en place sur l'utilisateur, lesdits moyens de fixation comportant au moins une attache apte à être déployée, cette attache étant maintenue initialement par des moyens de maintien dans un état rétracté et comportant une partie de préhension permettant à l'utilisateur de la déployer, les moyens de maintien comportant deux feuilles superposées prenant l'attache en sandwich.

On évite ainsi que l'attache soit exposée à un risque d'endommagement durant la manutention en machine.

L'invention permet de fabriquer la couche sans les bandes de réception précitées.

Le coût de la couche s'en trouve sensiblement réduit.

De plus, le fait de tirer sur les attaches présente un aspect ludique, susceptible de participer à l'éveil de l'enfant.

Dans une réalisation particulière, l'attache peut comporter un motif au recto et/ou verso (animaux, personnages, ...) n'apparaissant que lorsqu'elle est déployée.

De préférence, l'attache forme au moins deux plis à l'état rétracté.

Avantageusement, l'une des deux feuilles des moyens de maintien peut définir la surface extérieure de la couche et/ou l'une au moins des deux feuilles peut être constituée par une bande rapportée.

De tels moyens de maintien ne font pas intervenir d'autres éléments que ceux déjà conventionnellement utilisés dans la fabrication des couches à usage unique, ce qui permet une fabrication économique.

Les moyens de maintien peuvent encore être constitués par des panneaux latéraux prolongeant la couche sur les côtés à l'avant et/ou à l'arrière, rapportés par exemple sur le reste de la couche, et comprenant deux feuilles superposées, de préférence en non tissé.

De tels panneaux peuvent être rapportés sur le reste de la couche avec les attaches déjà placées à l'intérieur.

Les panneaux peuvent encore être réalisés par des prolongements d'un non tissé s'étendant sur toute la surface extérieure de la couche, doublée par un film imperméable, de polyéthylène par exemple, dans sa région centrale.

Avantageusement encore, des élastiques sont disposés entre les deux feuilles précitées, au moins dans la région d'entrejambes, améliorant ainsi le confort et l'étanchéité.

Avantageusement, l'attache comporte une partie collée ou soudée sur l'une au moins des deux feuilles.

L'attache peut ainsi comporter une partie collée ou soudée sur une face d'une feuille définissant par sa face opposée la surface extérieure de la couche ou comporter une partie collée ou soudée sur une bande, laquelle est rapportée sur une feuille définissant la surface extérieure de la couche.

Avantageusement, l'attache comporte un élément de fixation sur une face.

L'élément de fixation peut comporter des crochets, des boucles, un bouton pression, un adhésif ou un élément d'un système à emboîtement mécanique.

Lorsque l'élément de fixation comporte des crochets, il est avantageux qu'il soit recouvert au moins partiellement par un non tissé, l'attache étant à l'état rétracté, ce non tissé correspond au matériau de l'attache ou à celui de l'une des feuilles définissant le logement recevant l'attache.

On réduit ainsi le risque de glissement de la partie de préhension hors de son logement.

Bien entendu, on ne sort pas du cadre de l'invention lorsque l'élément de fixation est initialement totalement hors du logement recevant l'attache.

Dans une réalisation particulière, l'attache, une fois déployée, présente deux bords longitudinaux parallèles entre eux.

L'attache, une fois déployée, peut avoir par exemple une longueur comprise entre 5 et 15 cm et une largeur comprise entre 2 et 7 cm.

L'attache peut comporter entre la partie de préhension et la partie de fixation sur la couche une partie intermédiaire élastique.

Lorsque l'attache comporte une partie de fixation, une partie de préhension et une partie intermédiaire superposées au moins partiellement, la partie intermédiaire peut présenter une longueur supérieure à celle des parties préhension et de fixation.

L'invention a encore pour objet un procédé de fabrication d'une couche-culotte, comportant les étapes suivantes :
- pliage d'une bande suivant au moins deux lignes de pliage parallèles à une ligne longitudinale de la bande,
- fourniture d'un élément de fixation sur la bande, s'étendant sur une largeur inférieure à la largeur totale initiale de la bande,
- découpage de la bande pour constituer des attaches individuelles à l'état replié,
- fixation des attaches à l'état replié sur la couche.

Avantageusement, la couche comportant de manière conventionnelle une feuille qui est encollée sur toute sa longueur, excepté éventuellement dans une zone de dépose d'élastiques, on dépose les attaches individuelles à l'état replié, et l'on vient ensuite recouvrir la feuille extérieure et les attaches par une feuille intérieure ou par des bandes rapportées.

On procède ainsi en même temps à la fixation des attaches sur la couche et au collage de la feuille intérieure ou des bandes rapportées sur la feuille extérieure, ce qui permet de simplifier les étapes dans la fabrication de la couche et donc de gagner en productivité.

Avantageusement, la bande destinée à former les attaches individuelles est maintenue pliée en assemblant des parties superposées de celle-ci par soudure ou collage continu ou discontinu.

On peut, dans un mode de mise en oeuvre particulier, assembler les parties superposées de la bande localement, à des intervalles donnés dans le sens longitudinal, par exemple par soudure.

La présente invention sera mieux comprise à la lecture de la description détaillée qui va suivre, d'exemples non limitatifs, et au vu des dessins annexés, sur lesquels :
- la figure 1 représente une vue schématique et partielle de face d'une couche-culotte conforme à l'invention,
- la figure 2 est une coupe schématique suivant le trait de coupe II-II de la figure 1,
- la figure 3 est une coupe suivant le trait de coupe III-III de la figure 2,
- la figure 4 est une vue analogue à la figure 2 lorsque l'attache est déployée,
- la figure 5 est une vue analogue à la figure 2 d'une variante de l'invention,
- les figures 6 et 7 sont des vues en perspective, schématiques, de deux bandes pouvant servir à réaliser les attaches,
- les figures 8 à 11 illustrent différentes formes possibles d'attaches, et
- la figure 12 représente partiellement et de manière schématique une couche comprenant des panneaux latéraux rapportés.

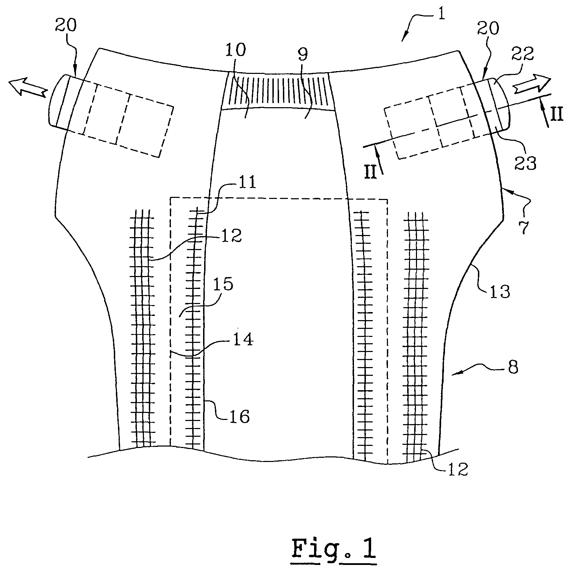

Sur la figure 1 est représentée partiellement la face intérieure d'une couche-culotte 1 conforme à l'invention.

Cette couche comporte une partie arrière 7, une partie d'entrejambes 8 et une partie avant non représentée.

Un élément absorbant 10 dont les côtés latéraux 14 ont été représentés en traits discontinus s'étend sur toute la partie d'entrejambes 8 et sur une portion de la partie arrière de la couche 1, de manière conventionnelle.

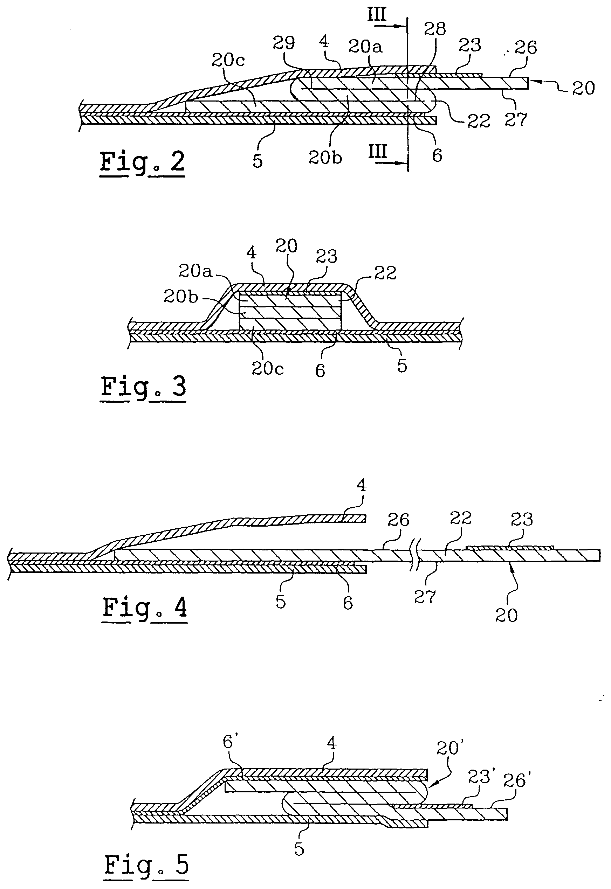

Comme on peut le voir sur la figure 2, la couche 1 comporte une feuille extérieure 5, qui définit la surface extérieure de la couche 1 et peut être constituée par exemple par un non tissé hydrophobe, perméable à l'air, ou en variante un complexe non tissé/film de polyéthylène ou de toute autre manière connue permettant de remplir la fonction recherchée.

Une feuille intérieure 9 hydrophile et perméable aux liquides vient recouvrir l'élément absorbant 10.

La couche 1 comporte sur sa longueur, de chaque côté de l'élément absorbant 10, des bandes 4 collées sur la feuille extérieure 5.

La partie d'entrejambes 8 comporte des découpes 13, de façon connue en soi.

Des élastiques 12 s'étendent sur au moins toute la partie d'entrejambes 8, étant pris en sandwich entre la feuille extérieure 5 et les bandes 4, de façon à constituer des bandes de jambes, de manière conventionnelle.

Des barrières fécales 15 sont formées au moyen des bandes 4 et comprennent au moins un élastique 11, également de manière conventionnelle.

Deux attaches 20 sont disposées de chaque côté de la partie arrière 7 de la couche-culotte 1, comme représenté sur la figure 1.

Leur disposition étant parfaitement symétrique, on n'a représenté en détail que l'attache 20 de droite sur les figures 2, 3 et 4.

L'attache 20 comprend, comme représenté sur la figure 2, une bande souple 22, pliée sur elle-même suivant deux pliures parallèles 28 et 29.

Cette bande est réalisée par exemple dans un non tissé.

L'attache 20 comporte une partie de fixation 20c collée sur la feuille extérieure 5, une partie de préhension 20a et une partie intermédiaire 20b reliant les parties de fixation 20c et de préhension 20a.

La partie de fixation 20c pourrait être collée à la fois à la feuille extérieure 5 et à la bande 4 correspondante.

Lorsque l'attache est à l'état replié, les parties 20a, 20b et 20c se superposent partiellement.

La partie intermédiaire 20b présente une longueur inférieure à celle des autres parties 20a et 20c.

La partie de préhension 20a s'étend partiellement hors du logement formé entre la feuille extérieure 5 et la bande correspondante 4.

La face 26 de l'attache 20 qui vient au contact de la face intérieure de la bande 4 comporte un élément de fixation 23, constitué dans l'exemple décrit par un moyen de fixation à crochets.

La face opposée 27 de l'attache 20 est collée par un film adhésif 6 à la face intérieure de la feuille extérieure 5.

L'élément de fixation 23 s'étend sur toute la largeur et sur une partie seulement de la longueur de la partie de préhension 20a, étant partiellement recouvert par la bande 4, comme on peut le voir sur les figures 1 et 2.

Lorsque la bande 4 est réalisée dans un non tissé, l'élément de fixation 23 s'accroche quelque peu à celle-ci, ce qui contribue au maintien en place de l'attache à l'état replié, en empêchant la partie de préhension 20a de glisser vers l'extérieur.

Les attaches 20 et 21 peuvent se déployer lorsque l'utilisateur exerce sur elles une traction dans les sens des flèches sur la figure 1.

Une fois déployées, elles peuvent être utilisées de manière conventionnelle pour assembler les parties avant et arrière de la couche, les éléments de fixation 23 coopérant avec une bande de réception à boucles, de façon connue en soi.

Bien entendu, l'invention n'est pas limitée à l'exemple qui vient d'être décrit.

On peut notamment modifier la manière dont l'attache est fixée sur la couche.

A titre d'exemple, on a représenté sur la figure 5 une attache 20' qui diffère de l'attache 20 précédemment décrite par le fait qu'elle comporte un élément de fixation 23' sur sa face 26' opposée à celle qui vient au contact de la feuille extérieure 5, et par le fait qu'elle est fixée par un film adhésif 6' sur la bande 4.

On va maintenant décrire un mode préféré de mise en place des attaches sur une couche.

Dans le cas des attaches 20, on commence par encoller la face intérieure de la feuille extérieure 5 selon deux bandes longitudinales dont la largeur est supérieure à la partie de fixation 20c, puis on dépose les élastiques 12 encollés dans une zone non encollée prévue à cet effet et les attaches à l'état replié et l'on recouvre la partie encollée par les bandes 4.

Ces dernières viennent recouvrir partiellement la partie de préhension 20a qui conserve une portion apparente, comme illustré à la figure 1.

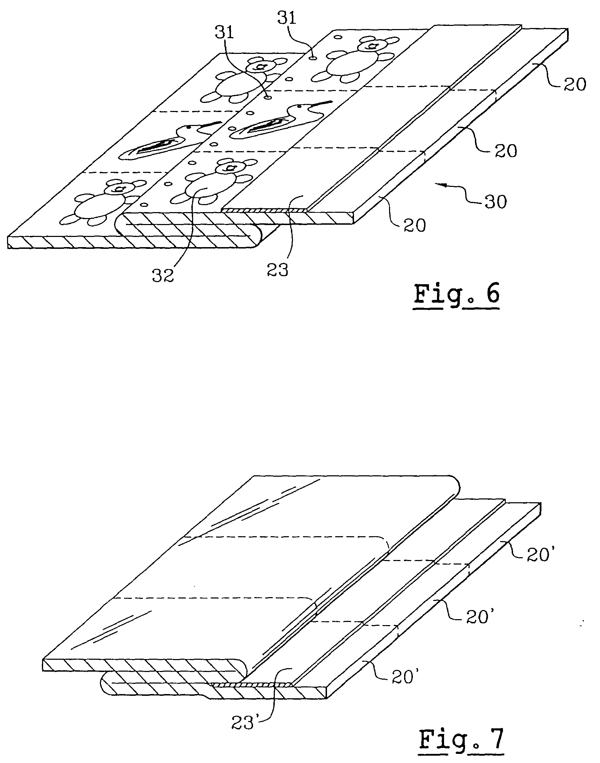

Les attaches 20 sont réalisées par découpage d'une bande 30 représentée sur la figure 6, qui présente en section transversale la même forme que les attaches à l'état replié.

Pour maintenir les parties superposées de la bande 30 assemblées les unes contre les autres, celle-ci comporte à des intervalles réguliers des points de soudure par ultrasons 31, créant des liens pouvant être facilement rompus lorsque l'utilisateur tire sur les attaches.

En variante, les parties superposées précitées peuvent être maintenues assemblées par tout système de collage ou de soudure, de manière intermittente ou continue.

Avantageusement, comme illustré sur la figure 6, la bande 30 est imprimée de telle sorte que chaque attache 20 comporte au moins un motif 32 qui, lorsque l'attache est à l'état rétracté, est caché par la bande 4 ou la feuille extérieure 5, et qui devient apparent lorsque l'attache 20 est à l'état déployé.

On a représenté sur la figure 7, une bande 30' servant à la réalisation des attaches 20'.

Bien entendu, l'invention n'est pas limitée aux exemples de réalisation qui viennent d'être décrits.

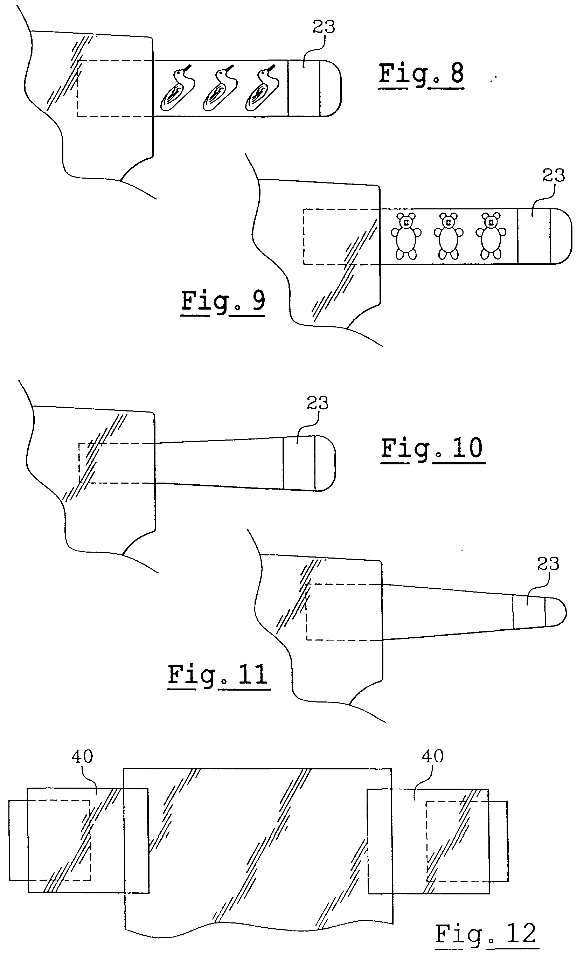

On peut notamment réaliser des attaches ayant des formes diverses, s'écartant de la forme de l'attache 20 représentée sur la figure 8.

On peut en particulier, comme illustré sur les figures 10 et 11, réaliser l'attache avec une largeur croissante ou décroissante en direction de son extrémité libre.

L'invention n'est pas limitée au maintien de l'attache à l'état replié dans un logement formé entre la feuille extérieure et une bande rapportée sur cette feuille.

On peut en particulier rapporter sur la feuille extérieure non pas une bande mais une feuille intérieure qui définirait toute la surface intérieure de la couche par exemple.

La partie intermédiaire des attaches peut être réalisée de manière à présenter une certaine élasticité.

On peut encore loger les attaches dans des panneaux latéraux 40 rapportés sur la couche, comme illustré sur la figure 12.

## Revendications

1. Couche-culotte (1) du type comportant des parties avant et arrière à assembler à l'aide de moyens de fixation pour maintenir la couche en place sur l'utilisateur, **caractérisée par le fait que** lesdits moyens de fixation comportent au moins une attache (20 ; 20') apte à être déployée, cette attache étant maintenue initialement par des moyens de maintien dans un état rétracté et comportant une partie de préhension (20a) permettant à l'utilisateur de la déployer, les moyens de maintien comportant deux feuilles (4 ; 5) superposées prenant l'attache (20 ; 20') en sandwich.

2. Couche-culotte (1) selon la revendication précédente, **caractérisée par le fait que** l'attache (20 ; 20') forme au moins deux plis (28 ; 29) à l'état rétracté.

3. Couche-culotte (1) selon l'une des revendications précédentes, **caractérisée par le fait que** l'une (5) des deux feuilles définit la surface extérieure de la couche.

4. Couche-culotte (1) selon la revendication 2 ou 3, **caractérisée par le fait que** l'une (4) au moins des deux feuilles est constituée par une bande rapportée.

5. Couche-culotte (1) selon l'une quelconque des revendications 2 à 4, **caractérisée par le fait que** des élastiques (12) sont disposés entre les deux feuilles (4 ; 5) au moins dans la région d'entrejambes.

6. Couche-culotte (1) selon l'une quelconque des revendications 2 à 5, **caractérisée par le fait que** l'attache (20 ; 20') comporte une partie (20c) collée ou soudée sur l'une au moins des deux feuilles.

7. Couche-culotte (1) selon l'une quelconque des revendications 2 à 5, **caractérisée par le fait que** l'attache comporte une partie collée ou soudée sur les deux feuilles (4 ; 5).

8. Couche-culotte (1) selon l'une quelconque des deux revendications immédiatement précédentes, **caractérisée par le fait que** l'attache (20) comporte une partie (20c) collée ou soudée sur une face d'une feuille (5) définissant par sa face opposée la surface extérieure de la couche.

9. Couche-culotte (1) selon la revendication 6 ou 7, **caractérisée par le fait que** l'attache (20') comporte une partie collée ou soudée sur une bande (4) rapportée sur une feuille (5) définissant la surface extérieure de la couche.

10. Couche-culotte (1) selon l'une quelconque des revendications 6 à 9, **caractérisée par le fait que** l'attache comporte entre la partie de préhension et la partie de fixation sur la couche, une partie intermédiaire élastique.

11. Couche-culotte (1) selon l'une quelconque des revendications précédentes, **caractérisée par le fait que** l'attache comporte un élément de fixation (23 ; 23') comprenant des crochets.

12. Couche-culotte (1) selon la revendication précédente, **caractérisée par le fait que** lorsque l'attache est à l'état replié, l'élément de fixation (23 ; 23') est au moins partiellement recouvert par un non tissé.

13. Couche-culotte (1) selon l'une quelconque des revendications 1 à 10, **caractérisée par le fait que** l'attache comporte un élément de fixation comprenant des bouches, un adhésif, un bouton-pression ou un élément d'un système à emboîtement mécanique.

14. Couche-culotte (1) selon l'une quelconque des revendications précédentes, **caractérisée par le fait que** l'attache comporte une partie de fixation, une partie de préhension et une partie intermédiaire au moins partiellement superposées, et **par le fait que** la partie intermédiaire (20b) présente une longueur supérieure à celle des parties de préhension (20a) et de fixation (20c).

15. Couche-culotte (1) selon l'une quelconque des revendications précédentes, **caractérisée par le fait que** l'attache (20 ; 20') une fois déployée a une longueur comprise entre 5 et 15 cm et une largeur comprise entre 2 et 7 cm.

16. Couche-culotte (1) selon l'une quelconque des revendications précédentes, **caractérisée par le fait qu'**elle comporte un motif (32) n'apparaissant que lorsqu'elle est déployée.

17. Couche-culotte (1) selon l'une quelconque des revendications 1 à 6, **caractérisée par le fait que** les attaches sont logées dans des panneaux latéraux (40) rapportés sur la couche.

18. Procédé de fabrication d'une couche-culotte (1) comportant les étapes suivantes :
- pliage d'une bande (30 ; 30') suivant au moins deux lignes de pliage parallèles à une ligne longitudinale de la bande,
- fourniture d'une bande de fixation (23 ; 23') sur la bande s'étendant sur une largeur inférieure à la largeur totale initiale de la bande (30 ; 30'),
- découpage de la bande (30 ; 30') pour constituer des attaches individuelles (20 ; 20') à l'état replié,
- fixation des attaches (20 ; 20) à l'état replié sur la couche.

19. Procédé selon la revendication immédiatement précédente, la couche comportant de manière conventionnelle une feuille extérieure (5), **caractérisé par le fait que** cette dernière est encollée sur toute sa longueur, excepté éventuellement dans une zone de dépose d'élastiques, et que l'on y dépose les attaches individuelles à l'état replié.

20. Procédé selon la revendication immédiatement précédente, **caractérisé par le fait que** l'on recouvre la feuille extérieure (5) et les attaches (20 ; 20') par une feuille intérieure ou par des bandes rapportées (4).

21. Procédé selon l'une quelconque des revendications 18 à 20, **caractérisé par le fait que** la bande (30 ; 30') destinée à recevoir les attaches individuelles (20 ; 20') est maintenue pliée en assemblant des parties superposées de la bande par soudure ou collage continu ou discontinu.
